# EUROPEAN PATENT SPECIFICATION

(11) **EP 4 124 251 B1**
(45) Date of publication and mention of the grant of the patent: **23.09.2026**
(21) Application number: 22772401.0
(22) Date of filing: 03.05.2022
(51) Int. Cl.: A24F 42/20, A24F 42/60, B05B 7/04, B05B 7/06, B05B 12/12, B65D 83/26, B65D 83/303, B65D 83/384, B65D 83/48, B65D 83/58, B65D 83/141, A24B 15/167, A61K 31/465, A61M 15/00

(54) **AEROSOL GENERATION DEVICE**
AEROSOLERZEUGUNGSVORRICHTUNG
DISPOSITIF DE GÉNÉRATION D'AÉROSOL

(30) Priority: 08.06.2021 KR 20210074297
(43) Date of publication of application: 01.02.2023
(73) Proprietor: KT&G Corporation, Daedeok-gu Daejeon 34337 (KR)
(72) Inventor: JEOUNG, Eun Mi, Daejeon 34128 (KR)
(74) Representative: Ter Meer Steinmeister & Partner
(86) International application number: PCT/KR2022/006291
(87) International publication number: WO 2022/260282

(56) References cited:
- WO-A1-2010/149280
- WO-A1-2017/167508
- CN-U- 212 940 910
- JP-A- 2019 513 012
- KR-A- 20070 022 640
- KR-A- 20150 060 964
- KR-B1- 100 971 178
- US-A1- 2019 335 814
- US-B2- 10 335 562

## Description

### TECHNICAL FIELD

Embodiments relate to an aerosol generating device, and more particularly, to an aerosol generating device that ejects an aerosol by using a canister in which nicotine and a propellant are stored.

### BACKGROUND ART

There is increasing demand for aerosol generating devices that generate aerosol in a non-combustion method as an alternative to burning cigarettes to generate aerosols. For example, the aerosol generating device is a device that generates aerosols in a non-combustion method from an aerosol generating material and supplies aerosols to a user or performs a function of generating an aerosol having a flavor by passing a vapor generated from the aerosol generating material through a fragrance medium.

For example, the non-combustion method of generating an aerosol may include a method of ejecting the aerosol by applying a high pressure to a liquid aerosol generating material to allow the liquid to pass through a nozzle. In another example, there may be a method of generating an aerosol by storing a propellant that is easily vaporized at a room temperature in a high-pressure canister together with an aerosol generating material and by allowing the propellant to be ejected out of the canister together with the aerosol generating material when an outlet of the canister is opened.

According to the non-combustion method, the aerosol generating device may not include a separate atomizer.

CN 212 940 910 U relates to an atomization device including a hollow housing having a cavity, wherein a liquid channel is arranged in the cavity, an atomizing nozzle at the distal end, a liquid delivery tube which has its liquid suction end located in a liquid reservoir and its liquid discharge end located in a liquid channel. The liquid channel includes a liquid chamber from which the liquid may flow out to the atomization nozzle.

WO 2017/167508 A1 relates to an atomizing assembly for an aerosol generating device. The atomizing assembly comprises a tubing section having an inlet end and an outlet end. The inlet end of the tubing section is configured to be connected to a liquid storage portion, and the outlet end of the tubing section is in fluid communication with an atomizing nozzle. The tubing section is configured for delivering a flow of liquid aerosol-forming substrate through the atomizing nozzle. The atomizing nozzle comprises an air channel for establishing an air flow through the nozzle. The air flow is mixed with the flow of liquid aerosol-forming substrate to enhance atomization of the flow of liquid aerosol-forming substrate delivered through the atomizing nozzle.

US 10 335 562 B2 relates to a pressurized metered dose dispenser for dispensing an aerosol formulation comprising particles of a medicament suspended in liquefied propellant, optionally in combination with one or more excipients, the dispenser comprising an aerosol container equipped with a metered dose valve, where a formulation chamber is defined in part by the internal walls of the container, and wherein the dispenser further comprises a porous, fluid permeable, particulate semi-permeable body located within the formulation chamber adjacent to the metered dose valve.

### DESCRIPTION OF EMBODIMENTS

### TECHNICAL PROBLEM

In an aerosol generating device using a method of ejecting an aerosol from a canister, smooth discharge of a liquid from the canister may be important in aerosol generating performance. In order for the liquid in the canister to be easily discharged regardless of conditions of the canister (for example, the amount of liquid remaining in the canister and a degree of inclination of the canister), the aerosol generating device may be designed to have a structure in which an outlet of the canister is arranged in a direction in which gravity acts (for example, a lower portion of the aerosol generating device).

In this case, a mouthpiece used when a user puffs on an aerosol generating device may also be under the aerosol generating device to be adjacent to the outlet of the canister. Accordingly, an aerosol generating device having a structure in which the mouthpiece is under the aerosol generating device from a user's point of view may be provided.

The aerosol generating device having the structure may provide unfamiliarity and discomfort to a user because a mouthpiece is in the direction facing the ground. Accordingly, an aerosol generating device having a structure in which a liquid in a canister is easily discharged but a mouthpiece is in an opposite direction to an outlet of the canister may be required.

Embodiments provide an aerosol generating device including an outlet arranged in a lower portion of a canister and a mouthpiece arranged in an upper portion of the aerosol generating device.

The technical problems of the present disclosure are not limited to the above-described description, and other technical problems may be clearly understood by one of ordinary skill in the art from the present specification and the attached drawings.

### SOLUTION TO PROBLEM

In order to overcome the drawbacks mentioned above, the present invention provides a device as defined in claim 1, wherein the dependent claims define advantageous embodiments thereof.

An aerosol generating device according to one embodiment may include a mouthpiece configured to come into contact with a mouth of a user, a canister storing a medium including nicotine and a propellant, and including an outlet which is on an opposite side of the mouthpiece and through which the medium flows out, a transfer tube having a first end portion connected to the outlet and a second end portion connected to the mouthpiece, and configured to receive the medium from the canister at the first end portion, a first valve arranged at the second end portion and configured to control discharge of the medium remaining in the transfer tube to the outside, and a nozzle configured to atomize the medium and eject an aerosol when the first valve is open.

### ADVANTAGEOUS EFFECTS OF DISCLOSURE

An aerosol generating device according to the embodiment described above includes an outlet of a canister which is downward to the aerosol generating device and a mouthpiece which is upward to the aerosol generating device, and thus, a liquid stored in the canister may be smoothly discharged, and user convenience may be obtained.

In addition, the aerosol generating device according to the embodiment described above includes a valve for opening and closing the outlet of the canister, and thus, the amount of medium discharged from the canister may be precisely controlled, and the amount of aerosol that is generated may be controlled according to needs of a user.

The effects of the present disclosure are not limited to the above effects, and effects that are not mentioned could be clearly understood by one of ordinary skill in the art from the present specification and the attached drawings.

### BRIEF DESCRIPTION OF DRAWINGS

FIG. 1A is a view schematically illustrating an aerosol generating device of a known embodiment.
FIG. 1B is a view schematically showing an aerosol generating device of another known embodiment.
FIG. 2 is a view schematically illustrating an aerosol generating device according to an embodiment.
FIG. 3 is a view schematically illustrating an aerosol generating device of another embodiment.
FIG. 4A is a partially enlarged view illustrating an aspect of an aerosol generating device according to an embodiment
FIG. 4B is a partial enlarged view illustrating another aspect of the aerosol generating device according to the embodiment.
FIG. 5 is a view schematically illustrating an aerosol generating device of another embodiment.
FIG. 6A is a view illustrating an air-atomizing nozzle according to an embodiment.
FIG. 6B is a view illustrating an air-atomizing nozzle according to another embodiment.

### MODE OF DISCLOSURE

With respect to the terms used to describe the various embodiments, general terms which are currently and widely used are selected in consideration of functions of structural elements in the various embodiments of the present disclosure. However, meanings of the terms can be changed according to intention, a judicial precedence, the appearance of new technology, and the like. In addition, in certain cases, a term which is not commonly used can be selected. In such a case, the meaning of the term will be described in detail at the corresponding portion in the description of the present disclosure. Therefore, the terms used in the various embodiments of the present disclosure should be defined based on the meanings of the terms and the descriptions provided herein.

In addition, unless explicitly described to the contrary, the word "comprise" and variations such as "comprises" or "comprising" will be understood to imply the inclusion of stated elements but not the exclusion of any other elements. In addition, the terms "-er", "-or", and "module" described in the specification mean units for processing at least one function and/or operation and can be implemented by hardware components or software components and combinations thereof.

As used herein, expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, or all of a, b, and c.

As will be described below, embodiments of the present disclosure will be described in detail with reference to the accompanying drawings so that those skilled in the art may easily implement the embodiments in the technical fields of the present disclosure. However, the embodiments of the present disclosure may be implemented in various different forms and are not limited to the embodiments described herein.

In addition, terms including an ordinal number such as 'first' or 'second' used in this specification may be used to describe various components, but the components should not be limited by the terms. The terms are used only for the purpose of distinguishing one component from another.

In addition, some of the components in the drawings may be illustrated with a somewhat exaggerated size or ratio. In addition, the components illustrated in some drawings may not be illustrated in other drawings.

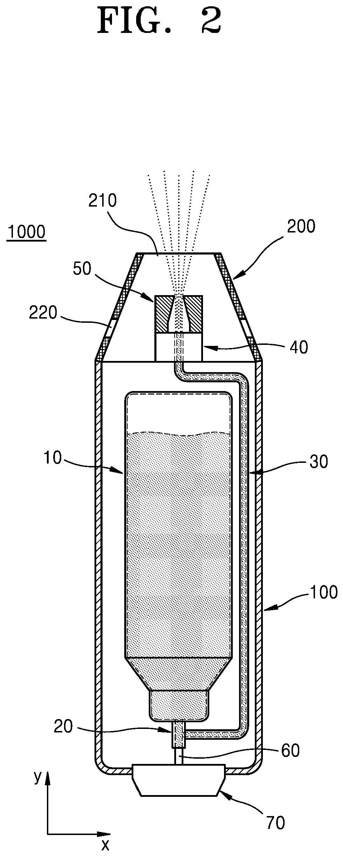

In addition, throughout the specification, a "longitudinal direction" of a component may indicate a direction in which the component extends along one axis thereof, in which case, one direction axis of the component may indicate a direction in which the component extends longer than another direction axis crossing the one-direction axis. For example, the longitudinal direction may indicate a direction parallel to another direction in which the transfer tube illustrated in FIG. 2 extends.

In addition, throughout the specification, an "upper portion" may indicate one end portion in the +y direction of the aerosol generating device illustrated in the drawings, and a "lower portion" may indicate one end portion in the -y direction. For example, in FIG. 2, a mouthpiece is in an upper portion of an aerosol generating device, and an outlet is in a lower portion of the aerosol generating device.

In addition, throughout the specification, "puff" refers to a user's inhalation, and the inhalation may refer to a situation in which something is drawn into a user's oral cavity, nasal cavity, or lungs through the user's mouth or nose.

In addition, throughout the specification, an "aerosol" refers to a material in which liquid and/or solid microparticles are dispersed in a gas. Aerosol may refer to a state in which vaporized particles generated from an aerosol generating material and air are mixed. For example, a phase of an aerosol generating material may be transformed into a gaseous phase through vaporization and/or sublimation. An aerosol may be generated by atomizing and releasing an aerosol generating material in a liquid and/or solid phase.

Throughout the specification, an "embodiment" is a certain distinction for easily describing the disclosure, and respective embodiments are not necessarily mutually exclusive. For example, configurations disclosed in one embodiment may be applied and/or implemented in other embodiments and may be modified and applied and/or implemented without departing from the scope of the disclosure.

In addition, the terms used in the present disclosure is for describing the embodiments and is not intented to limit the embodiments of the present disclosure. In the present disclosure, the singular also includes the plural unless otherwise specified.

Hereinafter, embodiments of the present disclosure will be described in detail with reference to the drawings.

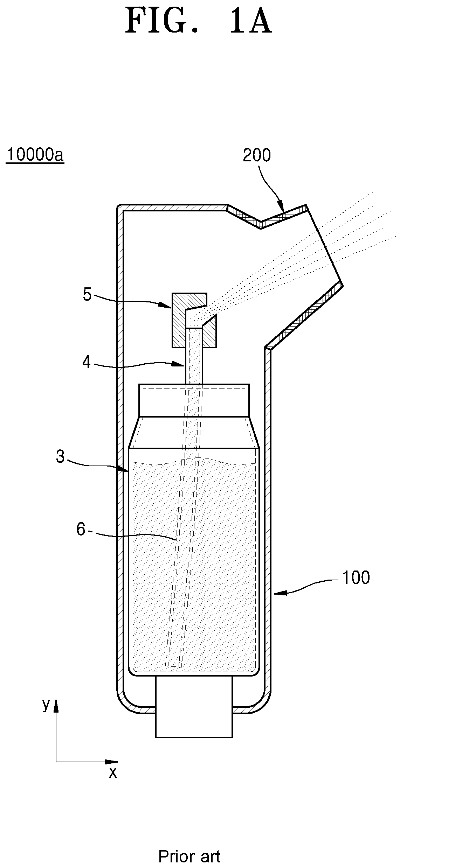

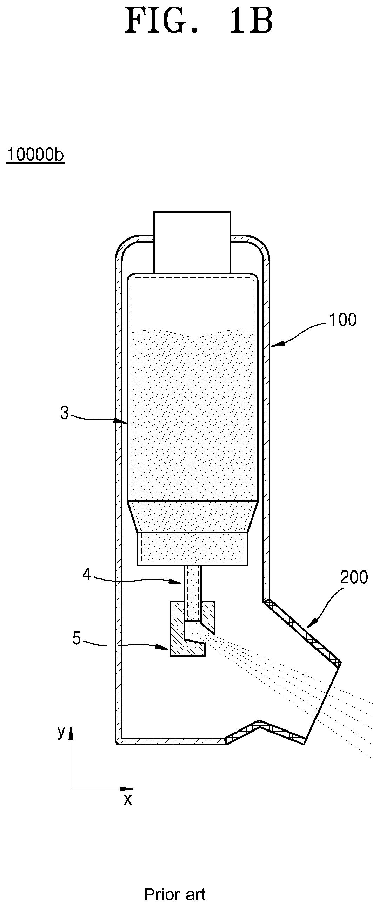

FIG. 1A is a view schematically illustrating an aerosol generating device according to a known embodiment, and FIG. 1B is a view schematically illustrating an aerosol generating device according to another known embodiment.

Referring to FIGS. 1A and 1B, aerosol generating devices 10000a and 10000b according to the known embodiments may each include a mouthpiece 200, a main body 100, a canister 3, an outlet 4, and a nozzle 5.

In the aerosol generating device 10000a according to the known embodiment illustrated in FIG. 1A, the mouthpiece 200 is at one end on an opposite side (for example, an upper portion of the aerosol generating device) of a direction in which gravity acts, and the outlet 4 of the canister 3 may also be located adjacent to the mouthpiece 200. Here, a direction opposite to the direction in which gravity acts may be, for example, a +y direction.

The canister 3 may include a propellant and an aerosol generating material. The inside of the canister 3 is maintained at a high pressure such that the propellant and the aerosol generating material included in the inside of the canister 3 may be maintained in a liquid state.

A material in the liquid state stored in the canister 3 having the structure illustrated in FIG. 1A may be affected by being discharged to the outside according to a condition of the canister 3. The condition of the canister 3 may include, for example, the remaining amount of an internal liquid, a position of an inner tube 6, an internal pressure, a degree of inclination to the ground, and so on, and the condition of the canister 3 may affect discharge of a liquid material stored in the canister 3.

That is, the canister 3, in which the outlet 4 is on the opposite side to the direction in which gravity acts, does not easily discharge the liquid therein, and thus, functionality and reliability of the aerosol generating device 10000a may be reduced. In order to solve this problem, a structure of the canister 3 illustrated in FIG. 1B may be proposed.

In the aerosol generating device 10000b according to another known embodiment illustrated in FIG. 1B, in order for a liquid material to be easily discharged to the outside of the canister 3, the outlet 4 may be at one end of the canister 3 in a direction in which gravity acts (for example, a lower portion of the aerosol generating device). Here, the direction in which gravity acts may be, for example, a -y direction.

As the outlet 4 of the canister 3 is in the lower portion of the canister 3, a liquid in the canister 3 may be easily discharged to the outside of the canister 3 without being affected by conditions of the canister 3 described above.

As the canister 3 has the structure illustrated in FIG. 1B, the mouthpiece 200 may also be at the lower portion of the aerosol generating device 10000b to be adjacent to the outlet 4 of the canister 3.

The aerosol generating device 10000b having the structure illustrated in FIG. 1B has an advantage that a liquid in the canister 3 is easily discharged compared to the aerosol generating device 10000a having the structure illustrated in FIG. 1A but may cause discomfort or unfamiliarity to a user because the mouthpiece 200 is in a direction toward the ground. Accordingly, there may be needed an aerosol generating device having a structure in which the outlet 4 of the canister 3 is in a lower portion of an aerosol generating device and the mouthpiece 200 is in an upper portion of the aerosol generating device.

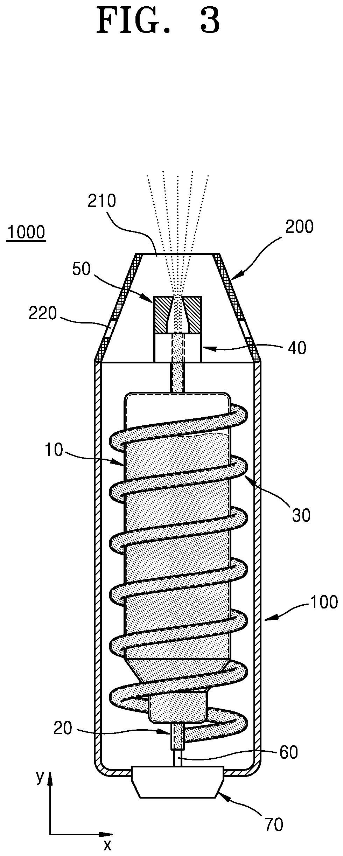

FIG. 2 is a view schematically illustrating an aerosol generating device according to an embodiment, and FIG. 3 is a view schematically illustrating an aerosol generating device according to another embodiment.

Referring to FIGS. FIGS. 2 and 3, an aerosol generating device 1000 according to the embodiment may include a mouthpiece 200, a main body 100, a canister 10, an outlet 20, a transfer tube 30, a first valve 40, a nozzle 50, a second valve 60, and a button 70. An arrangement of the transfer tube 30 is changed in the aerosol generating device 1000 illustrated in FIG. 3 compared to the aerosol generating device 1000 illustrated in FIG. 2.

A configuration of the aerosol generating device 1000 is not limited to the configurations illustrated in FIGS. 2 and 3. According to a design of the aerosol generating device 1000, some of the components illustrated in FIGS. 2 and 3 may be omitted or a new configuration may be added thereto, which may be understood by those skilled in the art related to the present embodiment.

The mouthpiece 200 may be at one end of the aerosol generating device 1000. For example, the aerosol generating device 1000 may be assembled by combining the main body 100 with the mouthpiece 200, and the mouthpiece 200 may be at one end in the +y direction, which is an upper portion of the aerosol generating device 1000.

The mouthpiece 200 is a portion to be inserted into a user's oral cavity. The mouthpiece 200 may include a discharge hole 210 for discharging an aerosol generated from the aerosol generating material in the canister 10 to the outside.

At least one region of the mouthpiece 200 may include an outdoor air inlet 220. The outdoor air inlet 220 is a passage through which external air may be introduced into the aerosol generating device 1000.

The external air introduced through the outdoor air inlet 220 may be introduced into a space in which an aerosol is generated. Accordingly, external air may be continuously introduced into the aerosol generating device 1000 through the outdoor air inlet 220, and thus, a user may perform continuous puff.

The canister 10 may store a medium comprising a propellant and an aerosol generating material. The canister 10 may be made of a material capable of endure a high pressure. For example, the canister 10 may be made of a metal or an alloy material but is not limited thereto.

The canister 10 may store a propellant in any one state among a liquid state, a solid state, a gaseous state, and a gel state, and an aerosol generating material.

The propellant may include at least one of hydrofluoroalkane (HFA) and chlorofluorocarbon (CFC), and the aerosol generating material may include a liquid composition. For example, the liquid composition may be a liquid including a tobacco material including a volatile tobacco flavor component or may also be a liquid including a non-tobacco material.

The liquid composition may include any one component among, for example, water, a solvent, ethanol, a plant extract, a fragrance, a flavoring agent, and a vitamin mixture, or a mixture of the components. The fragrance may include menthol, peppermint, spearmint oil, various fruit flavoring ingredients, and so on but is not limited thereto. The flavoring agent may include ingredients capable of providing a user with a variety of flavors or savors. The vitamin mixture may include a mixture of at least one of vitamin A, vitamin B, vitamin C, and vitamin E but is not limited thereto. In addition, the liquid composition may include an aerosol former such as glycerin and propylene glycol.

For example, the liquid composition may include a solution of glycerin and propylene glycol in any weight ratio, to which a nicotine salt is added. The liquid composition may also include two or more nicotine salts. The nicotine salt may be formed by adding a suitable acid including organic or inorganic acid to nicotine. The nicotine may include either naturally generated nicotine or synthetic nicotine and may have concentration of any suitable weight to the total solution weight of the liquid composition.

Acid for forming the nicotine salt may be appropriately selected by considering a blood nicotine absorption rate, an operating temperature of the aerosol generating device 1000, flavor or savor, solubility, and so on. For example, the acid for forming the nicotine salt may include benzoic acid, lactic acid, salicylic acid, lauric acid, sorbic acid, levulinic acid, pyruvic acid, formic acid, acetic acid, propionic acid, butyric acid, valeric acid, caproic acid, caprylic acid, capric acid, citric acid, myristic acid, palmitic acid, stearic acid, oleic acid, linoleic acid, linolenic acid, phenylacetic acid, tartaric acid, succinic acid, fumaric acid, gluconic acid, saccharinic acid, malonic acid, malic acid, a single acid selected from a group consisting of above acids, or a mixture of two or more acids selected from the group, but is not limited thereto.

According to one embodiment, the canister 10 may store a medium including nicotine and a propellant and may include the outlet 20 which is on an opposite side of the mouthpiece 200 and through which the medium flows out. For example, the canister 10 may include a first end portion facing the mouthpiece 200 and a second end portion opposite to the first end, and the outlet 20 may be at the second end portion.

A medium including a propellant and an aerosol generating material may be maintained in a liquid state in the canister 10 under a high pressure condition. When the outlet 20 of the canister 10 is open, the propellant included in the medium is vaporized or expanded, and thus, the medium may flow out or may be discharged to the outside of the canister 10.

The transfer tube 30 may receive the medium that flows out or is discharged from the canister 10. In one embodiment, the transfer tube 30 may include one end portion connected to the outlet 20 of the canister 10 and the other end portion connected to the mouthpiece 200, and the one end portion may receive a medium transferred from the canister 10.

The transfer tube 30 may be made of a material that may endure a pressure of the vaporized or expanded medium. For example, the transfer tube 30 may be made of a metal material (for example, aluminum or stainless steel) that may be maintained in a fixed shape. In another example, the transfer tube 30 may also be made of a material (for example, thermoplastic polyurethane) with excellent food hygiene.

The medium transferred from the canister 10 to the transfer tube 30 may temporarily stay in the transfer tube 30 before being discharged to the outside of the aerosol generating device 1000. For example, the first valve 40 in the other end portion of the transfer tube 30 may control that the medium transferred to the transfer tube 30 is discharged to the outside of the aerosol generating device 1000. The first valve 40 permits or blocks the medium remaining in the transfer tube 30 from being discharged to the outside of the aerosol generating device 1000, and a more detailed description thereof will be described below.

According to one embodiment, a diameter and a length of the transfer tube 30 may be designed based on a user's aerosol puff amount. For example, the diameter and length of the transfer tube 30 may be designed based on the amount of medium required for one puff or one smoking by a user.

As at least one of the diameter and length of the transfer tube 30 increases, a volume of the transfer tube 30 increases, and thus, the greatest amount of a medium that may stay in the transfer tube 30 may increase. That is, by adjusting the diameter and length of the transfer tube 30, the amount of aerosol generated by the aerosol generating device 1000 per puff or per smoking may be changed.

In this way, the transfer tube 30 may connect the outlet 20 and the mouthpiece 200 of the canister 10 to each other, which are separated from each other, to transfer the medium inside the aerosol generating device 1000, and the amount of aerosol generated by the aerosol generating device 1000 may be adjusted or changed for a user according to the design of the transfer tube 30.

According to the embodiment illustrated in FIG. 2, the transfer tube 30 may extend along an outer peripheral surface of the canister 10. For example, the transfer tube 30 may extend in a straight line or a curved line from the one end portion connected to the outlet 20 to the other end portion connected to the mouthpiece 200 along the outer peripheral surface in a state of being separated from the outer peripheral surface of the canister 10 by a preset distance. In another example, the transfer tube 30 may also extend in a straight line or a curved line from the one end portion connected to the outlet 20 to the other end portion connected to the mouthpiece 200 along the outer peripheral surface in a state of being in contact with the outer peripheral surface of the canister 10.

According to another embodiment illustrated in FIG. 3, the transfer tube 30 may be spirally wound along the outer peripheral surface of the canister 10. Here, "winding spirally along an outer peripheral surface of a canister" means that the transfer tube 30 is wound around the canister 10 along the outer peripheral surface of the canister 10 in a shape corresponding to the shape of the canister 10. For example, the canister 10 may have a cylindrical shape, a triangular prism shape, a square prism shape, or a polygonal prism shape, and the transfer tube 30 may be wound in a shape corresponding to the shape of the canister 10, for example, a circular spiral, a triangular spiral, a square spiral, or a polygonal spiral.

**In** one embodiment, the canister 10 and the transfer tube 30 may be integrated with each other. For example, the transfer tube 30 may be wound along the outer peripheral surface of the canister 10 to pressurize the outer peripheral surface of the canister 10. **In** another example, at least a part of the transfer tube 30 may be bonded to the outer peripheral surface of the canister 10. Specifically, the transfer tube 30 may be bonded to the canister 10 by welding, an adhesive, or so on such that the canister 10 may be integrated with the transfer tube 30.

As the transfer tube 30 has a spiral structure, capacity of the transfer tube 30 is greatly increased in the limited space in the main body 100, and the transfer tube 30 may occupy an appropriate space, and thus, components in the aerosol generating device 1000 may be efficiently arranged.

**In** addition, a position of the transfer tube 30 wound around the canister 10 or integrated with the canister 10 may be fixed when an external impact or so on is applied thereto, and thus, structural stability may be increased.

In another embodiment, the transfer tube 30 may be formed in various shapes and may be arranged in a part in the main body 100 of the aerosol generating device 1000. For example, the transfer tube 30 may be designed to have a preset diameter and a preset length as described above and may be arranged in a part in the main body 100 including a wound portion, a curled portion, or a tortuous portion. Accordingly, a space (or a volume) occupied by the transfer tube 30 in the main body 100 may be reduced, and thus, space utilization inside the main body 100 may be increased.

The first valve 40 may be arranged at the other end portion of the transfer tube 30 to control discharge of a medium remaining in the transfer tube 30 to the outside. For example, the first valve 40 may prevent a medium transferred from the canister 10 to the transfer tube 30 from leaking from the transfer tube 30 to the outside of the aerosol generating device 1000 by closing the other end portion of the transfer tube 30 when a user does not use the aerosol generating device 1000, and may allow the medium transferred to the transfer tube 30 to be discharged to the outside of the aerosol generating device 1000 by opening the other end portion of the transfer tube 30 when the user uses the aerosol generating device 1000.

In another example, the first valve 40 may close a hole (or a vent) of the nozzle 50 to be described below when a user does not use the aerosol generating device 1000 and may open the hole of the nozzle 50 when the user uses the aerosol generating device 1000, and thereby, a medium may be discharged to the outside of the aerosol generating device 1000. That is, when the first valve 40 is open, a medium remaining in the transfer tube 30 may pass through the nozzle 50 to be ejected to the outside.

According to one embodiment, the first valve 40 may be opened based on a user's puff. For example, the first valve 40 may be opened when a user puffs on the aerosol generating device 1000 and may be closed when the user does not puff on the aerosol generating device 1000. That is, the aerosol generating device 1000 according to the embodiment may adjust generation of an aerosol based on a user's puff.

The first valve 40 may include a mechanical operation valve that opens above a preset threshold pressure and closes below the preset threshold pressure. The mechanically operation valve may not be opened by a pressure of a medium staying in the transfer tube 30 and may be opened only when a negative pressure is applied to the other end portion of the transfer tube 30 according to the user's puff on the aerosol generating device 1000.

In one embodiment, the first valve 40 may include a needle valve (or a "pintle"). The needle valve may have a shape (for example, a cone) having one end closed at an acute angle and may be displaced according to a change in pressure due to a user's puff on the aerosol generating device 1000 to open or close a hole thereof. For example, the other end portion of the transfer tube 30 may be opened or closed based on an upward movement or a downward movement of the needle valve. In another example, a gap of the hole of the nozzle 50, which will be described later, may be opened or closed based on the upward movement or the downward movement of the needle valve.

The type of the first valve 40 is not limited to the examples described above, and the first valve 40 may include an electromagnetic operation valve that operates based on a user's input. The electromagnetic operation valve may include, for example, a solenoid valve. When a user's puff or a user's operation is detected, the solenoid valve generates a magnetic force to open a flow path in the solenoid valve.

When the first valve 40 is opened, the nozzle 50 may atomize a medium that is discharged to eject an aerosol. The nozzle 50 may expand a fluid by increasing a flow rate of the fluid passing therethrough and decreasing a pressure thereof. For example, the nozzle 50 may have a shape in which a cross-sectional area thereof decreases along a direction in which a medium is discharged. As a medium passing through the nozzle 50 expands, sizes of particles are atomized and uniformized, and thereby, an aerosol may be ejected from an outlet of the nozzle 50.

A passage of the nozzle 50 may have a preset length. Here, the preset length may indicate a length enough to allow a medium (or an aerosol) passing through the passage inside the nozzle 50 to be atomized into a sufficiently small size (for example, 10 µm in diameter). For example, the nozzle 50 may extend in a curved line from the first valve 40 by a preset length.

In one embodiment, the passage of the nozzle 50 may include a curved portion. For example, the passage of the nozzle 50 may include a portion for changing a traveling direction of a medium. In another example, the passage of the nozzle 50 may also tortuously extend to have a preset length inside the nozzle 50.

As the length of the passage of the nozzle 50 increases, a travel distance of a medium inside the nozzle 50 may increase. Accordingly, an average size of particles of an aerosol ejected from the nozzle 50 may be further reduced, and thus, a user may more smoothly inhale the aerosol.

According to one embodiment, the nozzle 50 may include an air-atomizing nozzle that includes an outdoor air passage to mix an aerosol with outdoor air and eject the mixture. A more detailed description of the air-atomizing nozzle will be made below.

As described above, the outlet 20 of the canister 10 may be at the other end on a lower side of the aerosol generating device 1000, and the mouthpiece 200 may be at one end on an upper side of the aerosol generating device 1000. Accordingly, problems of the aerosol generating devices 10000a and 10000b according to the known embodiments described with reference to FIGS. 1A and 1B may be solved.

In one embodiment, the nozzle 50 and the mouthpiece 200 may be arranged in the same direction as each other. The discharge hole 210 included in the mouthpiece 200 may be disposed in a direction parallel to the direction in which the nozzle 50 ejects the medium.

For example, the nozzle 50 may be disposed to eject the medium in the +y direction, and the discharge hole 210 of the mouthpiece 200 may also be arranged in the +y direction. In another example, the nozzle 50 may be arranged to eject a medium in one direction between the +x direction and the +y direction, and the discharge hole 210 of the mouthpiece 200 may also be arranged in the same direction as the nozzle 50.

By matching a direction of the nozzle 50 to a direction of the discharge hole 210, an aerosol may be more smoothly transferred through the mouthpiece 200 to a user using the aerosol generating device 1000.

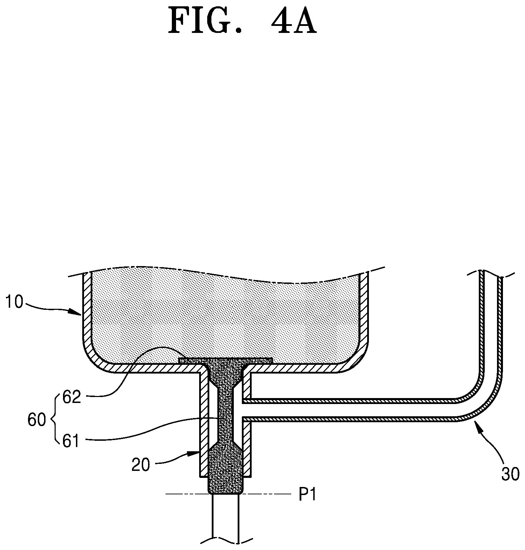

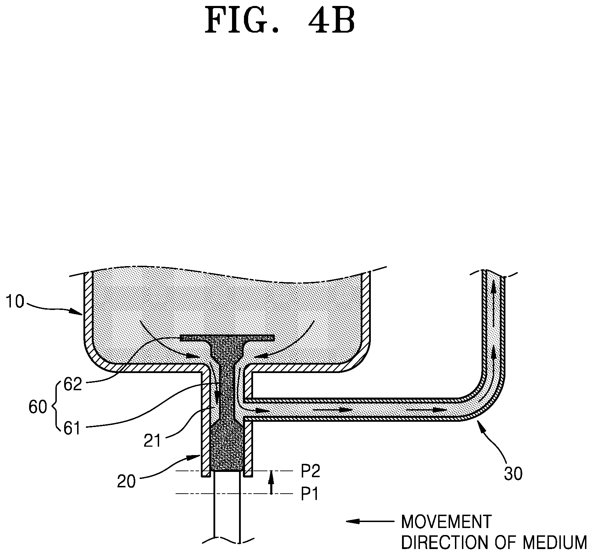

Hereinafter, an operation of the second valve 60 will be described in more detail with reference to FIGS. 4A and 4B.

FIG. 4A is a partially enlarged view illustrating an aspect of an aerosol generating device according to an embodiment, and FIG. 4B is a partially enlarged view illustrating another aspect of the aerosol generating device according to the embodiment.

Referring to FIGS. 4A and 4B, the aerosol generating device 1000 according to the embodiment may further include the second valve 60 that controls opening and closing of the outlet 20 to control transfer of a medium stored in the canister 10 to the transfer tube 30. The second valve 60 may open or close the outlet 20 of the canister 10 while moving between, for example a first position P1 and a second position P2.

In one aspect, the second valve 60 may close the outlet 20 of the canister 10 when located in the first position P1. The inside of the canister 10 is in a relatively high pressure state, and thus, a medium in the canister 10 tends to move to the outside, but an outward movement of the medium may be blocked by the second valve 60 that closes the outlet 20.

According to one embodiment, the second valve 60 may include a body portion 61 for controlling opening and closing of the outlet 20 and a locking portion 62 for limiting a movement range of the body portion 61. The locking portion 62 may be caught on the inside of the canister 10 to prevent the body portion 61 from being separated from the canister 10 by an internal pressure of the canister 10. That is, the locking portion 62 may prevent the outlet 20 from being opened by the second valve 60 due to a pressure inside the canister 10 causing the second valve 60 to deviate from a movement range.

In addition, the second valve 60 may further include an elastic portion (not illustrated) for maintaining a closed state of the outlet 20 when the aerosol generating device 1000 is not in use.

In another aspect, the second valve 60 may open the outlet 20 of the canister 10 when located in the second position P2. The second valve 60 may move from the first position P1 to the second position P2 when one side thereof is pressurized. When the second valve 60 moves to the second position P2, a gap 21 may be made such that the outlet 20 communicates with the transfer tube 30, and the medium stored in the canister 10 may be transferred to the transfer tube 30 through the gap 21.

An opening/closing mechanism of the outlet 20 by the second valve 60 is not limited to the method described above, and it may be understood by those skilled in the art related to the present embodiment that various opening/closing mechanisms such as a rotary opening/closing mechanism may be applied to the aerosol generating device 1000 according to the embodiment.

In addition, the canister 10, the transfer tube 30, and the second valve 60 may form an integral unit, and when any one of the canister 10, the transfer tube 30, and the second valve 60 has reduced durability or a failure, components of the integral unit may be replaced individually, or all the components may be replaced.

According to one embodiment, the second valve 60 may be operated by a user, and the medium stored in the canister 10 may be transferred to the transfer tube 30 in response to an operation of a user for the second valve 60 to open the outlet 20 . For example, a user may pressurize the second valve 60 to move from the first position P1 to the second position P2.

Specifically, the aerosol generating device 1000 according to the embodiment may further include a button 70 for controlling an operation of the second valve 60. Here, the button 70 may be implemented by a physical button or a capacitive touch sensor.

For example, the operation of the second valve 60 may be mechanically controlled according to an operation of the button 70. When the button 70 connected to the second valve 60 is pressed, one end of the second valve 60 is pressurized, and thus the outlet 20 may be opened.

In another example, the operation of the second valve 60 may be electrically adjusted according to the operation of the button 70. Specifically, when the button 70 is pressed, an electromagnet for applying a magnetic force to the second valve 60 may be electrically connected to a battery for supplying a current to the electromagnet. That is, the button 70 may serve as a switch for connecting the battery to the electromagnet. When the button 70 is pressed, a current may be applied to the electromagnet, and the electromagnet may generate a magnetic field that applies a magnetic force to the second valve 60 such that one end of the second valve 60 is pressurized, and then the outlet 20 may be opened.

In this way, the button 70 may be physically or electrically connected to the second valve 60, and when a user operates the button 70, the button 70 may move from the first position P1 to the second position P2 or from the second position P2 to the first position P1 in order to adjust opening and closing of the outlet 20 of the canister 10.

In one embodiment, the button 70 may be in a housing that forms an exterior of the aerosol generating device 1000. Here, the housing may indicate an external case for protecting a main body thereof from an external impact. For example, the button 70 may be at one end on a lower side of the aerosol generating device 1000. In another example, the button 70 may be at the outer peripheral surface of the aerosol generating device 1000.

A user may conveniently control an operation of the second valve 60 by pressing the button 70 while holding the aerosol generating device 1000.

Because the operation of the second valve 60 is adjusted based on the operation of the button 70, the amount of medium transferred to the transfer tube 30 may be determined based on at least one of an operation time and an operation strength of the button 70.

The medium transferred to the transfer tube 30 may not be discharged directly to the outside of the aerosol generating device 1000 and may be discharged to the outside of the aerosol generating device 1000 under the condition that the first valve 40 is open, and thus, a user may directly operate the button 70 such that a desired amount of medium is transferred from the canister 10 to the transfer tube 30.

That is, a user may preliminarily determine the amount of aerosol to be generated before puffing on the aerosol generating device 1000 by directly controlling opening and closing of the second valve 60. Accordingly, a user may directly control the amount of aerosol ejected to the outside of the aerosol generating device 1000 when the first valve 40 is opened.

Inside the aerosol generating device 1000 according to the embodiments described above, a movement process of a medium may be divided into a movement process from the canister 10 to the transfer tube 30 and a movement process from the transfer tube 30 to the nozzle 50, and the first and second valves 40 and 60 for controlling the two movement processes may be operated or controlled by a user.

For example, a user may open the second valve 60 before puffing on the aerosol generating device 1000 to allow a preset amount of medium to move from the canister 10 to the transfer tube 30, and by puffing on the aerosol generating device 1000 to open the first valve 40 when generating an aerosol, the medium remaining in the transfer tube 30 may move to the nozzle 50.

**In** this way, the aerosol generating device 1000 includes the first valve 40 and the second valve 60, and thus, a possibility of leakage of the medium in the aerosol generating device 1000 may be doubly blocked, and a user may have the desired amount of aerosol generated at desired timing.

The aerosol generating device 1000 according to the embodiment described above may have cross-sectional shape, such as a circular shape, an oval shape, a square shape, a rectangular shape, or a polygonal shape, in a direction crossing a longitudinal direction of a main body thereof. However, the cross-sectional shape of the aerosol generating device 1000 is not limited to the shapes described above, and the aerosol generating device 1000 is not limited to a linearly extending structure when extending in the longitudinal direction. For example, the cross-sectional shape of the aerosol generating device 1000 may be curved in a streamline shape for a user's hand grip or may be bent at a preset angle in a specific region to extend long, and the cross-sectional shape of the aerosol generating device 1000 may change in the longitudinal direction.

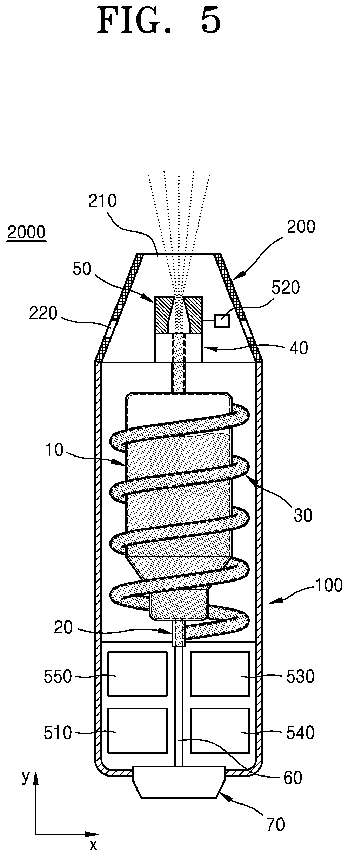

FIG. 5 is a view schematically illustrating an aerosol generating device according to another embodiment.

Referring to FIG. 5, an aerosol generating device 2000 according to another embodiment may include a mouthpiece 200, a main body 100, a canister 10, an outlet 20, a transfer tube 30, a first valve 40, a nozzle 50, a second valve 60, a button 70, a battery 510, a sensor 520, a user interface 530, a memory 540, and a processor 550.

The aerosol generating device 2000 illustrated in FIG. 5 may be different from the aerosol generating device 2000 illustrated in FIG. 3 in that some hardware components are added thereto, and descriptions overlapping the content described in FIG. 3 are omitted, and the added components will be mainly described.

The hardware components may be arranged on a lower side of the aerosol generating device 2000 but is not limited to the configuration illustrated in FIG. 5. It may be understood by those skilled in the art related to the present embodiment that an arrangement of the configuration of the aerosol generating device 2000 may be changed according to a design of the aerosol generating device 2000.

The battery 510 may supply power that is used to operate the aerosol generating device 2000. In addition, the battery 510 may supply power required for operations of other hardware components included in the aerosol generating device 2000, that is, the sensor 520, the user interface 530, the memory 540, and the processor 550. The battery 510 may include a rechargeable battery or a disposable battery.

For example, the battery 510 may include a nickel-based battery (for example, a nickel-metal hydride battery or a nickel-cadmium battery) or a lithium-based battery (for example, a lithium-cobalt battery, a lithium-phosphate battery, a lithium titanate battery, a lithium-ion battery, or a lithium-polymer battery). However, the type of the battery 510 that may be used in the aerosol generating device 2000 is not limited to the battery described above. The battery 510 may include an alkaline battery or a manganese battery as needed.

The aerosol generating device 2000 may include at least one sensor 520. A result detected by the at least one sensor 520 may be transmitted to the processor 550, and according to the detected result, the processor 550 may control the aerosol generating device 2000 to perform various functions such as displaying a notification.

For example, the at least one sensor 520 may include a puff detection sensor. The puff detection sensor may detect a user's puff based on at least one of a change in a flow rate, a change in pressure, and detection of a sound of an externally introduced airflow. The puff detection sensor may detect a start timing and an end timing of a user's puff, and the processor 550 may determine a puff period and a non-puff period according to the detected start timing and end timing of the puff.

According to one embodiment, the aerosol generating device 2000 may further include a puff detection sensor for detecting a user's puff, and the first valve 40 may operate based on a result detected by the puff detection sensor. For example, the first valve 40 may include a solenoid valve, and the solenoid valve may operate based on the result detected by the puff detection sensor.

The solenoid valve may generate a magnetic force based on a user's puff detected by the puff detection sensor. For example, a result detected by the puff detection sensor may be transferred to the processor 550, and the processor 550 may control a current supplied from the battery 510 to the solenoid valve based on the detected result.

In one embodiment, the inside of the solenoid valve may include a moving body that is movable by an applied magnetic force. When a user's puff is not detected, a magnetic force is not applied to the moving body, and thus, the moving body may be maintained in a position in which a flow path inside the solenoid valve is closed, and when the user's puff is detected, a magnetic force is applied to the moving body, and thus, the moving body may move from a position in which the flow path is closed to a position in which the flow path is opened.

In addition, the at least one sensor 520 may include a user input sensor. The user input sensor may be a sensor, which receives a user's input, such as a switch, a physical button, or a touch sensor. For example, the touch sensor may include a capacitive sensor that may detect a user's input by detecting a change in capacitance generated when the user touches a preset region formed of a metal material. The processor 550 may determine whether there is a user's input by comparing values before and after the change in capacitance received from the capacitive sensor. When the value before and after the change in capacitance exceeds a preset threshold, the processor 550 may determine that there is a user's input.

In addition, the at least one sensor 520 may include a motion sensor. Information on movement of the aerosol generating device 2000, such as inclination, movement speed, and acceleration of the aerosol generating device 2000, may be acquired by the motion sensor. For example, the motion sensor may detect information on a moving state of the aerosol generating device 2000 is moving, a stationary state of the aerosol generating device 2000, a state in which the aerosol generating device 2000 is inclined at an angle in a preset range for puff, and a state in which the aerosol generating device 2000 is inclined at an angle different from an angle formed during a puff operation between respective puff operations. The motion sensor may detect motion information of the aerosol generating device 2000 by using various methods known in the art. For example, the motion sensor may include an acceleration sensor that may detect acceleration in three directions of an x-axis, a y-axis, and a z-axis, and a gyro sensor that may detect angular velocity in the three directions.

The memory 540 may include hardware for storing various data processed by the aerosol generating device 2000, and the memory 540 may store data processed by the processor 550 and data to be processed thereby. The memory 540 may include various types of memory, for example, random access memory (RAM) such as dynamic random access memory (DRAM) or static random access memory (SRAM), read-only memory (ROM), electrically erasable programmable read-only memory (EEPROM), and so on.

The memory 540 may store an operating time, a greatest number of puffs, a current number of puffs of the aerosol generating device 2000, and data on a user's smoking pattern.

The processor 550 controls all operations of the aerosol generating device 2000. The processor 550 may also be implemented by an array of a plurality of logic gates or may also be implemented by a combination of a general-purpose microprocessor 550 and a memory unit that stores a program executable by the microprocessor 550. In addition, it may be understood by those skilled in the art that the processor 550 may also be implemented by other types of hardware.

The processor 550 may analyze a result detected by the at least one sensor 520 and control subsequent processing to be performed. For example, the processor 550 may control opening and closing of the first valve 40 and/or the second valve 60 based on a result detected by at least one sensor.

The embodiment may also be implemented in the form of a recording medium including instructions executable by a computer such as a program module to be executed by a computer. Computer-readable media may be any available media accessible by a computer and include both volatile and nonvolatile media and removable and non-removable media. In addition, the computer-readable media may include both computer storage media and communication media. The computer storage media may include both volatile and nonvolatile media and removable and non-removable media implemented by any method or technology for storing information such as computer readable instructions, data structures, program modules or other data. The communication media typically include computer readable instructions, other data in modulated data signals such as program modules, or other transmission mechanisms, and include any information transfer media.

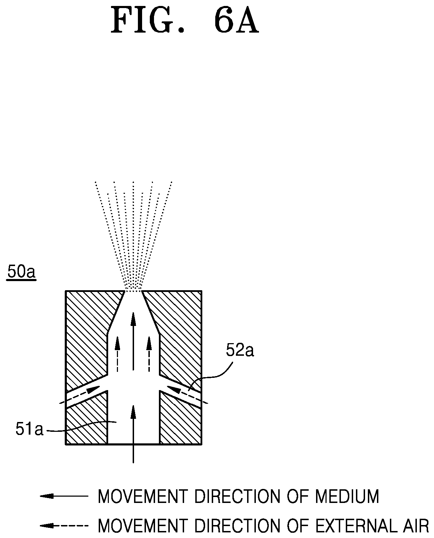

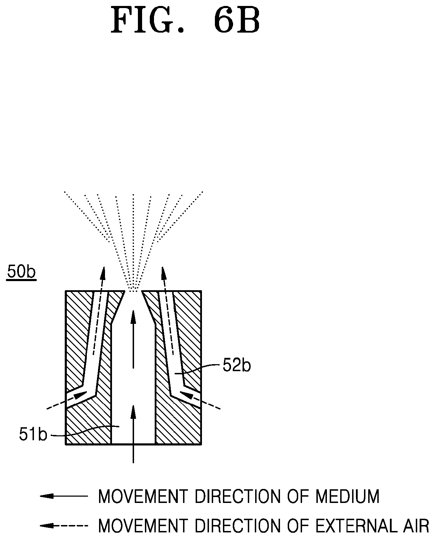

FIG. 6A is a view illustrating an air-atomizing nozzle according to an embodiment, and FIG. 6B is a view illustrating an air-atomizing nozzle according to another embodiment.

Referring to FIGS. 6A and 6B, air-atomizing nozzles 50a and 50b according to the embodiments may respectively include outdoor air passages 52a and 52b through which external air is introduced. For example, the air-atomizing nozzles 50a and 50b may respectively include at least one outdoor air passage 52a and at least one outdoor air passage 52b through which external air is introduced, and a medium and external air may be mixed on the inside or outside of the nozzles 50a and 50b.

In one embodiment, the air-atomizing nozzle 50a may include at least one medium passage 51a and the outdoor air passage 52a, and the medium passage 51a may be in fluid communication with the outdoor air passage 52a on the inside of the nozzle 50a. Accordingly, a medium moving to the outside of the nozzle 50a through the medium passage 51a may be mixed or collide with the external air introduced into the nozzle 50a in one region inside the nozzle 50a.

In another embodiment, the air-atomizing nozzle 50b may include at least one medium passage 51b and the outdoor air passage 52b, and the medium passage 51b and the outdoor air passage 52b may extend to the end of the nozzle 50b from which an aerosol is ejected, and the medium passage 51b may be in fluid communication with the outside air passage 52b on the outside of the end of the nozzle 50b from which the aerosol is ejected. Accordingly, a medium and external air discharged through the nozzle 50b may be mixed or collide with external air on the outside of the end of the nozzle 50b.

As such, media passing through the air-atomizing nozzles 50a and 50b may be mixed or collide with external air on the inside or outside of the nozzles 50a and 50b, and thus, an aerosol having smaller particles may be effectively generated.

## Claims

1. An aerosol generating device (1000; 2000) comprising:
a mouthpiece (200) configured to come into contact with a mouth of a user;
a canister (10) storing a medium including nicotine and a propellant, and including a first end portion facing the mouthpiece (200), a second end portion opposite to the first end, and an outlet (20) at the second end portion, wherein the outlet (20) is on an opposite side of the mouthpiece (200) and through the outlet (20) the medium flows out;
a transfer tube (30) having a first end portion connected to the outlet (20) and a second end portion connected to the mouthpiece (200), and configured to receive the medium from the canister (10) at the first end portion;
a first valve (40) arranged at the second end portion and configured to control discharge of the medium remaining in the transfer tube (30) to the outside; and
a nozzle (50) configured to atomize the medium and eject an aerosol when the first valve (40) is open.

2. The aerosol generating device (2000) of claim 1, wherein the first valve (40) operates based on puff of the user.

3. The aerosol generating device (2000) of claim 2, further comprising a puff detection sensor (520) configured to detect the puff of the user,
wherein the first valve (40) operates based on a result detected by the puff detection sensor (520).

4. The aerosol generating device (1000; 2000) of claim 1, further comprising a second valve (60) configured to control transfer of the medium stored in the canister (10) to the transfer tube (30) by adjusting opening and closing of the outlet (20).

5. The aerosol generating device (1000; 2000) of claim 4, wherein
the second valve (60) is operated by the user, and
the medium stored in the canister (10) is transferred to the transfer tube (30) in response to an operation of the user for the second valve (60) to open the outlet (20).

6. The aerosol generating device (1000; 2000) of claim 5, wherein the second valve (60) is movable between a first position in which the outlet (20) is closed and a second position in which the outlet (20) is opened.

7. The aerosol generating device (1000; 2000) of claim 5, further comprising a button (70) configured to control the operation of the second valve (60),
wherein an amount of the medium transferred to the transfer tube (30) is determined based on at least one of an operation time and an operation strength of the button (70).

8. The aerosol generating device (1000; 2000) of claim 1, wherein a diameter and a length of the transfer tube (30) is designed based on an aerosol puff amount of the user.

9. The aerosol generating device (1000; 2000) of claim 1, wherein the transfer tube (30) is spirally wound on an outer peripheral surface of the canister (10).

10. The aerosol generating device (1000; 2000) of claim 1, wherein at least a part of the transfer tube (30) is in contact with the outer peripheral surface of the canister (10).

11. The aerosol generating device (1000; 2000) of claim 1, wherein the nozzle (50) includes an air-atomizing nozzle including an outdoor air passage through which external air is introduced.

12. The aerosol generating device (1000; 2000) of claim 1, wherein the nozzle (50) includes a passage through which the medium passes, and the passage has a curved portion.

13. The aerosol generating device (1000; 2000) of claim 1, wherein
the mouthpiece (200) has a discharge hole (210) for discharging the aerosol to the outside, and
the discharge hole (210) is arranged in a direction parallel to another direction in which the nozzle (50) ejects the medium.

## Patentansprüche

1. Aerosolerzeugungsvorrichtung (1000; 2000), die Folgendes umfasst:
ein Mundstück (200), das konfiguriert ist, mit dem Mund eines Benutzers in Kontakt zu gelangen;
einen Behälter (10), der ein Medium speichert, das Nikotin und ein Treibmittel enthält, und einen ersten Endabschnitt, der dem Mundstück (200) zugewandt ist, einen zweiten Endabschnitt, der dem ersten Ende gegenüberliegt, und einen Auslass (20) am zweiten Endabschnitt aufweist, wobei sich der Auslass (20) auf einer dem Mundstück (200) gegenüberliegenden Seite befindet und das Medium durch den Auslass (20) ausströmt;
einen Übertragungsschlauch (30), der einen ersten Endabschnitt, der mit dem Auslass (20) verbunden ist, und einen zweiten Endabschnitt, der mit dem Mundstück (200) verbunden ist, aufweist und konfiguriert ist, das Medium aus dem Behälter (10) am ersten Endabschnitt aufzunehmen;
ein erstes Ventil (40), das am zweiten Endabschnitt angeordnet ist und konfiguriert ist, das Auslassen nach außen des Mediums, das im Übertragungsschlauch (30) zurückbleibt, zu steuern; und
eine Düse (50), die konfiguriert ist, das Medium zu zerstäuben und ein Aerosol auszustoßen, wenn das erste Ventil (40) offen ist.

2. Aerosolerzeugungsvorrichtung (2000) nach Anspruch 1, wobei das erste Ventil (40) auf Basis eines Zugs des Benutzers arbeitet.

3. Aerosolerzeugungsvorrichtung (2000) nach Anspruch 2, die ferner einen Zugdetektionssensor (520) umfasst, der konfiguriert ist, den Zug des Benutzers zu detektieren,
wobei das erste Ventil (40) auf Basis eines Ergebnisses arbeitet, das durch den Zugdetektionssensor (520) detektiert wird.

4. Aerosolerzeugungsvorrichtung (1000; 2000) nach Anspruch 1, die ferner ein zweites Ventil (60) umfasst, das konfiguriert ist, die Übertragung des im Behälter (10) gespeicherten Mediums zum Übertragunsschlauch (30) durch Einstellen des Öffnens und Schließens des Auslasses (20) zu steuern.

5. Aerosolerzeugungsvorrichtung (1000; 2000) nach Anspruch 4, wobei das zweite Ventil (60) durch den Benutzer betätigt wird, und
das im Behälter (10) gespeicherte Medium als Antwort auf eine Betätigung des Benutzers, damit das zweite Ventil (60) den Auslass (20) öffnet, zum Übertragungsschlauch (30) übertragen wird.

6. Aerosolerzeugungsvorrichtung (1000; 2000) nach Anspruch 5, wobei das zweite Ventil (60) zwischen einer ersten Position, in der der Auslass (20) geschlossen ist, und einer zweiten Position, in der der Auslass (20) geöffnet ist, beweglich ist.

7. Aerosolerzeugungsvorrichtung (1000; 2000) nach Anspruch 5, die ferner einen Knopf (70) aufweist, der konfiguriert ist, die Betätigung des zweiten Ventils (60) zu steuern,
wobei eine Menge des Mediums, die zum Übertragungsschlauch (30) übertragen wird, auf Basis einer Betätigungszeit und/oder einer Betätigungsstärke des Knopfs (70) bestimmt wird.

8. Aerosolerzeugungsvorrichtung (1000; 2000) nach Anspruch 1, wobei ein Durchmesser und eine Länge des Übertragungsschlauchs (30) auf Basis einer Aerosolzugmenge des Benutzers entworfen werden.

9. Aerosolerzeugungsvorrichtung (1000; 2000) nach Anspruch 1, wobei der Übertragungsschlauch (30) schraubenlinienförmig auf eine Außenumfangsfläche des Behälters (10) gewickelt ist.

10. Aerosolerzeugungsvorrichtung (1000; 2000) nach Anspruch 1, wobei sich zumindest ein Teil des Übertragungsschlauchs (30) mit der Außenumfangsfläche des Behälters (10) in Kontakt befindet.

11. Aerosolerzeugungsvorrichtung (1000; 2000) nach Anspruch 1, wobei die Düse (50) eine Luftzerstäubungsdüse enthält, die einen Außenluftkanal enthält, durch den Außenluft eingeleitet wird.

12. Aerosolerzeugungsvorrichtung (1000; 2000) nach Anspruch 1, wobei die Düse (50) einen Kanal enthält, durch den das Medium strömt, und der Kanal einen gekrümmten Abschnitt aufweist.

13. Aerosolerzeugungsvorrichtung (1000; 2000) nach Anspruch 1, wobei
das Mundstück (200) ein Auslassloch (210) zum Auslassen des Aerosols nach außen aufweist, und
das Auslassloch (210) in einer Richtung parallel zu einer weiteren Richtung, in der die Düse (50) das Medium ausstößt, angeordnet ist.

## Revendications

1. Dispositif de production d'aérosol (1000 ; 2000) comportant :
un embout buccal (200) configuré pour venir en contact avec une bouche d'un utilisateur ;
une cartouche (10) stockant un milieu incluant de la nicotine et un agent propulseur, et incluant une première portion d'extrémité dirigée vers l'embout buccal (200), une seconde partie d'extrémité opposée à la première extrémité, et une sortie (20) sur la seconde portion d'extrémité, dans lequel la sortie (20) est sur un côté opposé de l'embout buccal (200) et par l'intermédiaire de la sortie (20), le milieu s'écoule vers l'extérieur ;
un tube de transfert (30) ayant une première portion d'extrémité reliée à la sortie (20) et une seconde portion d'extrémité reliée à l'embout buccal (200), et étant configuré pour recevoir le milieu provenant du récipient (10) sur la première portion d'extrémité ;
une première soupape (40) disposée sur la seconde portion d'extrémité et configurée pour commander une évacuation du milieu restant dans le tube de transfert (30) vers l'extérieur ; et
une buse (50) configurée pour atomiser le milieu et éjecter un aérosol lorsque la première soupape (40) est ouverte.

2. Dispositif de production d'aérosol (2000) selon la revendication 1, dans lequel la première soupape (40) fonctionne sur la base d'une bouffée de l'utilisateur.

3. Dispositif de production d'aérosol (2000) selon la revendication 2, comportant en outre un capteur de détection de bouffée (520) configuré pour détecter la bouffée de l'utilisateur,
dans lequel la première soupape (40) fonctionne sur la base d'un résultat détecté par le capteur de détection de bouffée (520).

4. Dispositif de production d'aérosol (1000 ; 2000) selon la revendication 1, comportant en outre une seconde soupape (60) configurée pour commander un transfert du milieu stocké dans la cartouche (10) vers le tube de transfert (30) en réglant une ouverture et une fermeture de la sortie (20).

5. Dispositif de production d'aérosol (1000 ; 2000) selon la revendication 4, dans lequel
la seconde soupape (60) est actionnée par l'utilisateur, et
le milieu stocké dans la cartouche (10) est transféré vers le tube de transfert (30) en réponse à un actionnement de l'utilisateur pour que la seconde soupape (60) ouvre la sortie (20).

6. Dispositif de production d'aérosol (1000 ; 2000) selon la revendication 5, dans lequel la seconde soupape (60) est mobile entre une première position dans laquelle la sortie (20) est fermée, et une seconde position dans laquelle la sortie (20) est ouverte.

7. Dispositif de production d'aérosol (1000 ; 2000) selon la revendication 5, comportant en outre un bouton (70) configuré pour commander l'actionnement de la seconde soupape (60),
dans lequel une quantité du milieu transféré vers le tube de transfert (30) est déterminée sur la base d'au moins un élément parmi un temps d'actionnement et une intensité d'actionnement du bouton (70).

8. Dispositif de production d'aérosol (1000 ; 2000) selon la revendication 1, dans lequel un diamètre et une longueur du tube de transfert (30) sont conçus sur la base d'une quantité de bouffée d'aérosol de l'utilisateur.

9. Dispositif de production d'aérosol (1000 ; 2000) selon la revendication 1, dans lequel le tube de transfert (30) est enroulé en spirale sur une surface périphérique extérieure de la cartouche (10).

10. Dispositif de production d'aérosol (1000 ; 2000) selon la revendication 1, dans lequel au moins une partie du tube de transfert (30) est en contact avec la surface périphérique extérieure de la cartouche (10).

11. Dispositif de production d'aérosol (1000 ; 2000) selon la revendication 1, dans lequel la buse (50) inclut une buse d'atomisation d'air incluant un passage d'air extérieur à travers lequel de l'air externe est introduit.

12. Dispositif de production d'aérosol (1000 ; 2000) selon la revendication 1, dans lequel la buse (50) inclut un passage par lequel passe le milieu, et le passage a une partie incurvée.

13. Dispositif de production d'aérosol (1000 ; 2000) selon la revendication 1, dans lequel
l'embout buccal (200) comporte un trou d'évacuation (210) pour évacuer l'aérosol vers l'extérieur, et
le trou d'évacuation (210) est disposé dans une direction parallèle à une autre direction dans laquelle la buse (50) éjecte le milieu.
